# EUROPEAN PATENT APPLICATION

(11) **EP 4 424 820 A1**
(43) Date of publication of application: **04.09.2024**
(21) Application number: 23160049.5
(22) Date of filing: 03.03.2023
(51) Int. Cl.: C12N 9/00

(54) **DESIGNED SYNZIP PROTEIN DOMAINS FOR GENERATING ARTIFICIAL NONRIBOSOMAL PEPTIDE SYNTHETASES AND HYBRIDS THEREOF**

(71) Applicant: Max-Planck-Gesellschaft zur Förderung der Wissenschaften e.V., 80539 München (DE); Myria Biosciences AG, 4058 Basel (CH)
(72) Inventor: The designation of the inventor has not yet been filed
(74) Representative: Kuttenkeuler, David

(57) **Abstract**

The invention is based on newly derivatized protein interaction adaptor protein sequences based on SYNZIP protein domains. The improved SYNZIP domain variants of the invention were designed to be suitable for interconnecting domains of non-ribosomal peptide synthetases (NRPS) or domains of polyketide synthases (PKS), such as for generating NRPS-PKS hybrid complexes. The invention provides the SYNZIP derivative sequences, NRPS domains containing them, NRPS domain libraries thereof, as well as methods for the production and use in peptide design, screening and production.

## Description

### FIELD OF THE INVENTION

The invention is based on newly derivatized protein interaction adaptor protein sequences based on SYNZIP protein domains. The improved SYNZIP domain variants of the invention were designed to be suitable for interconnecting domains of non-ribosomal peptide synthetases (NRPS) or domains of polyketide synthases (PKS), such as for generating NRPS-PKS hybrid complexes. The invention provides the SYNZIP derivative sequences, NRPS domains containing them, NRPS domain libraries thereof, as well as methods for the production and use in peptide design, screening and production.

### DESCRIPTION

Synthetic biology (SynBio) seeks to create or redesign systems and cells with molecular biology tools and genome editing technologies to produce novel biological parts, such as molecules, materials or cells. For example, synthetic biology holds the potential to bio-synthesize drugs, chemicals and bio-fuels more sustainably and cost-effectively than with traditional methods. Multimodular enzyme complexes, such as polyketide synthases and non-ribosomal peptide synthetases (NRPSs), are often subject of SynBio - because they produce a variety of valuable chemicals, or pharmaceutically relevant peptides. Engineering these biosynthetic assembly lines can produce artificial polyketides, non-ribosomal peptides (NRPs), or hybrids thereof with new or improved properties.

Recently, we have focused in particular on developing more efficient SynBio methods for engineering modular NRPSs (type I NRPS), which has been in its infancy for decades. By introducing the exchange Unit (XU) and the XU Condensation Domain (XUC) concepts, we provided the necessary means to enable more reproducible, predictable and robust engineering than before. The XU concept, for example, leverages a fusion point located between the NRPS's condensation (C) and adenylation (A) domains within the structurally flexible region of the C-A interdomain linker. A domains in NRPSs are responsible for ATP-dependent selection and activation of specific amino acids (AAs), while C-domains catalyze peptide-bond formation between two AA residues. Together with the T domain, which transports the activated AA from the A-domain to the C-domain, they form the core domains of a canonical minimal NRPS module (C-A-T). NRPSs usually consist of not just one but several sequentially repeating modules, each responsible for the incorporation and optional functional group modification of a specific AA. Commonly, the number of modules in a NRPS corresponds directly to the number of AAs incorporated into the associated NRP. Of note, Dell et al., re-categorized ribosome-independent peptide assembly systems into five groups (type I, II, III, IV and V) and assigned NRPSs to type I and type II. Type I and II are distinguished by their modular or non-modular/freestanding character, with modular NRPSs classified as type I and freestanding NRPSs as type II NRPSs. Iterative systems, such as the enniatin or rhabdopeptide producing NRPSs that reuse individual modules, no longer represent a separate group, but are assigned to type I NRPSs.

Despite the progress made, assembly line engineering remained difficult and time consuming due to their mere size and repetitive nature. Traditional NRPS cloning and engineering often requires elaborated cloning strategies such as yeast cloning, LLHR (Linear plus linear homologous recombination-mediated recombineering) or ExoCET (Exonuclease Combined with RecET recombination) which are frequently accompanied with technical problems and limitations.7 Therefore, we established a new synthetic type of NRPS (type S), that enables easier and faster cloning by splitting large one protein modular NRPSs into two or three smaller and independently expressible subunits.

SYNZIPs are heterospecific synthetic coiled coils that enable controlled protein interaction and are used in synthetic biology. Coiled coils are generally composed of two, three or four amphipathic 20-50 AS-long α helices which form an intertwined left-handed supercoil. They are structural motifs of many proteins and also occur, for example, in the leucine zipper regions of the human bZIP transcription factor. Coiled coils are characterised by a heptade pattern which carry hydrophobic AS at positions 1 and 4, while electrostatic AS usually occur at positions 5 and 7. In an α-helical secondary structure, these hydrophobic AS interact with one another and form a narrow hydrophobic interface.

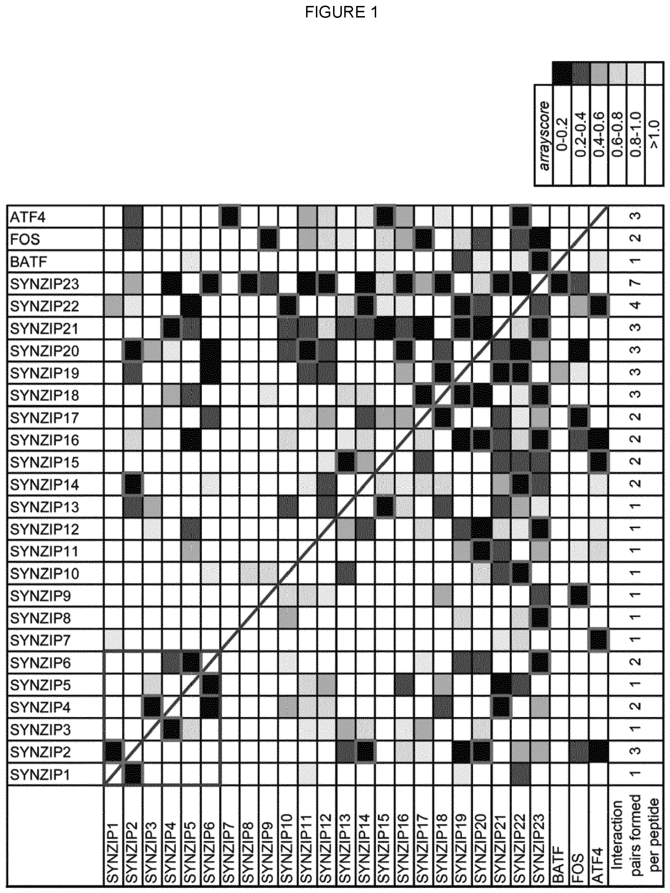

SYNZIPs were originally developed for the heterospecific interaction with leucine zipper regions of human bZIP transcription factors (TF). For this purpose, 48 artificial peptides were constructed on the basis of a computer, which were subsequently investigated with regard to their interaction with these peptides. In a further study, on the other hand, the interaction of the peptides with one another was also tested. To this end, Reinke et al. carried out a protein-microarray assay in which all 48 artificial as well as 7 further coiled-coils of human bZIPs were tested against one another (Fig. 1). From the results of the assay, 27 pairs, 23 synthetic ones (namely SYNZIP 1-23) and three human bZIP structures were selected which showed a strong heterospecific and, at the same time, low homospecific interaction. As indicated in Fig. 1, the peptides are involved in at least one to a maximum of seven interactions. Furthermore, it was concluded from the Asn-Asn pairing on the a-a' positions that most pairs must be parallel heterodimers (Reinke et al., 2010, see Fig. 1).

The international publication WO 2019/138117 describes a system for assembling and modifying NRPS. The system uses novel, precisely defined building blocks (units) which comprise condensation subdomains. This strategy enables the efficient combination of assemblies, which are referred to as exchange Units (XU2.0), irrespective of their naturally occurring specificity for the subsequent NRPS adenylation domain. The system of WO 2019/138117 enables the simple assembly of NRPS with an activity for the synthesis of a peptide with any amino acid sequence, without restrictions due to naturally occurring NRPS units. The system also makes it possible to exchange natural NRPS building blocks with the XU2.0 according to the invention, as a result of which modified peptides are produced. Although the system allows a simple combination of XU2.0 units, it still requires the expression of the assembled NRPS protein in an open reading frame (ORF). This leads to problems, especially in the case of longer NRPs.

The international publication WO 2021/094462 discloses a system for expressing nonribosomal peptide synthetases (NRPSs), polyketide synthases (PKS) or NRPS/PKS hybrid synth(et)ases. NRPS, PKS or hybrids thereof as large multi-domain proteins or multi-domain complexes which are assembled post-translationally via protein-protein interactions, introduced in a targeted manner using SYNZIP sequences, to form multi-enzyme complexes.

Thus, it is an object of the invention to provide improved SYNZIP sequences that are adjusted to the assembly of NRPS/PKS domains post-translationally via SYNZIP mediated protein-protein interactions. This object is solved by the present invention, which is summarized by the following aspects.

### BRIEF DESCRIPTION OF THE INVENTION

Generally, and by way of brief description, the main aspects of the present invention can be described as follows:

In **a first aspect,** the invention pertains to a synthetic fusion protein, comprising sequence elements (a), (b) and optionally (c), wherein:
**(a)** Is a non-ribosomal peptide synthetase (NRPS) or polyketide synthetase (PKS) amino acid sequence;
**(b)** Is a modified SYNZIP amino acid sequence; and
**(c)** Is a linker amino acid sequence which is in between (a) and (b).

In **a second aspect,** the invention pertains to an assembly system of synthetic NRPS or PKS complexes, comprising at least two synthetic fusion protein of the first aspect, wherein at least one of the at least two fusion protein is a donor synthetic fusion protein and is in N- to C-terminal direction (a), optionally (c), and (b); and wherein at least one of the at least two fusion proteins is an acceptor synthetic fusion protein and is in N- to C-terminal direction (b), optionally (c), and (a).

In **a third aspect,** the invention pertains to a synthetic NRPS or PKS complex, wherein at least two NRPS or PKS domains are connected via a non-covalent interaction between two modified SYNZIP amino acid sequences.

In **a fourth aspect,** the invention pertains to a nucleic acid encoding a synthetic fusion protein of the first aspect.

In **a fifth aspect,** the invention pertains to a library of nucleic acid sequences, wherein the library comprises two or more nucleic acids, and wherein the two or more nucleic acids comprised in the library encode the assembly system of synthetic NRPS or PKS complexes of any one of the preceding aspects.

In **a sixth aspect,** the invention pertains to a method of assembling a complete or partial synthetic NRPS or PKS, the method comprising bringing into contact, or expressing together, at least a donor synthetic fusion protein and an acceptor synthetic fusion protein of any one of the preceding claims.

### DETAILED DESCRIPTION OF THE INVENTION

In the following, the elements of the invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine two or more of the explicitly described embodiments or which combine the one or more of the explicitly described embodiments with any number of the disclosed and/or preferred elements. Furthermore, any permutations and combinations of all described elements in this application should be considered disclosed by the description of the present application unless the context indicates otherwise.

The invention will be described using in the terms which are defined as follows:

The term "partial domain" or "partial C or C/E domain", or similar terms, refers to a nucleic acid sequence encoding an NRPS or PKS domain or a protein sequence of such domain which is incomplete (not in full length). In this context, the term is to be understood as meaning that, compared with the full-length domain, the partial domain has a contiguous proportion of at least 20%, preferably 30% or 40% or more, of a successive sequence of the full-length domain. A partial domain therefore has a very high degree of sequence identity (90% and more) with respect to a coherent portion (at least 20%, preferably 30% or 40% or more) of the sequence of the full-length domain. For example, the expression describes a C or C/E domain sequence which does not comprise both donor and acceptor sites of an NRPS-C or C/E domain. Partial domains of NRPS have, for example, a sequence length of 100 or more, 150 or more, or about 200 amino acids.

"Compilation" refers to a number of domains. A plurality of NRPS/PKS assemblies includes a complete NRPS-PKS. One or more polypeptides may comprise a module. Module combinations then catalyse longer peptides in combination. In one example, a module may comprise a C domain (condensation domain), an A domain (adenylation domain), and a peptidyl carrier protein domain.

Further structural information on A domains, C domains, didomains, domain-domain interfaces and complete modules can be found at Conti et al. (1997), Sundlov et al. (2013), Samel et al. (2007), Tanovic et al. (2008), Strieker and Marahiel (2010), Mitchell et al. (2012) and Tan et al. (2015).

"Initiation module" means an N-terminal module that can transfer a first monomer to another module (e.g., an extension or final module). In some cases, the further module is not the second module, but one of the C-terminally following modules (for example in the case of the Nocardicin NRPS). In the case of an NRPS, an initiation module comprises, for example, an A (adenylation) domain and a PCP (peptidyl carrier protein) or a T (thiolation) domain. The initiation module can also contain a starter C domain and/or an E domain (epimerisation domain). With a PKS, a possible initiation module consists of an AT domain (acetyl transferase) and an ACP domain (acyl carrier protein). Initiation modules are preferably located at the amino terminus of a polypeptide of the first module of an "assembly series". Each assembly series preferably contains an initiation module.

The term "extension module" or "elongation module" refers to a module that adds a donor monomer to an acceptor monomer or an acceptor polymer, thereby extending the peptide chain. An elongation module may comprise a C (condensation), Cy (heterocyclisation), E, C/E, MT (methyltransferase), A-MT (combined adenylation and methylation domain), Ox (oxidase) or Re (reductase) domain; an A domain; or a T domain. An elongation domain may further comprise additional E, Re, DH (dehydration), MT, NMet (N-methylation), AMT (aminotransferase) or Cy domains. In addition, an elongation module could be of PKS origin and could comprise the respective domains (ketosynthase (KS), acyltransferase (AT), ketoreductase (KR), dehydratase (DH), enoyl reductase (ER, thiolation (T)).

"Termination module" refers to a module that releases or decouples the molecule (e.g., an NRP, a PK, or combinations thereof) from the assembly series. The molecule can be released, for example, by hydrolysis or cyclisation. Terminating modules may comprise a TE (thioesterase), Cterm (terminal C domain) or Re domain. The termination module is preferably located at the carboxy terminus of an NRPS or PKS polypeptide. The termination module may further comprise additional enzymatic activities (e.g., oligomerase activity).

"Domain" means a polypeptide sequence or a fragment of a larger polypeptide sequence having one or more specific enzymatic activities (i.e., C/E domains have a C and an E function in a domain or another conserved function (i.e., as a binding function for an ACP or T domain). Thus, a single polypeptide may comprise multiple domains. Multiple domains can form modules. Examples of domains are C (condensation), Cy (heterocyclisation), A (adenylation), T (thiolation), TE (thioesterase), E (epimerisation), C/E (condensation/epimerisation), MT (methyltransferase). Ox (oxidase), Re (reductase), KS (ketosynthase), AT (acyltransferase), KR (ketoreductase), DH (dehydratase) and ER (enoyl reductase).

"Non-ribosomally synthesised peptide", "non-ribosomal peptide" or 'NRP' refers to any polypeptide that is not produced by a ribosome. NRPs may be linear, cyclic or branched, and may contain proteinogenic, natural or non-natural amino acids, or any combination thereof. The NRPs include peptides which are produced in a type of assembly line or series (= modular character of the enzyme system, which enables the gradual addition of building blocks to the end product).

"Polyketide" refers to a compound which comprises a plurality of ketone units.

"Non-ribosomal peptide synthetase" or "non-ribosomal peptide synthetase" or "NRPS" refers to a polypeptide or a series of interacting polypeptides which produce a non-ribosomal peptide and can thus catalyse the formation of peptide bonds without ribosomal components. "Polyketide synthase" (PKS) refers to a polypeptide or a series of polypeptides that produce a polyketide without ribosomal components.

"Non-ribosomal peptide synthetase/polyketide synthase hybrid" or "hybrid of non-ribosomal peptide synthetases and polyketide synthases" or "NRPS/PKS hybrid" or "hybrid of NRPS and PKS" or "hybrid of PKS and NRPS" and other corresponding expressions refer to an enzyme system comprising any domains or modules of NRPS and PKS. Such hybrids catalyse the synthesis of natural hybrid substances.

"Change in a structure" means any change in a chemical (e.g., covalent or non-covalent) bond compared to a reference structure.

"Mutation" refers to a change in the nucleic acid sequence, so that the amino acid sequence encoded by the nucleic acid sequence has at least one amino acid change in comparison with the naturally occurring sequence. The mutation can, without limitation, be an insertion, deletion, frame shift mutation or a missense mutation. This term also describes a protein which is encoded by the mutated nucleic acid sequence.

A "variant" is a polypeptide or polynucleotide having at least 10%, 20%, 30%, 40%, 50%, 60%, 70%, 80%, 90%, 95% or 99% sequence identity to a reference sequence. The sequence identity is typically measured using sequence analysis software (for example, sequence analysis software package from the Genetics Computer Group, Biotechnology Center of the University of Wisconsin, 1710 University Avenue, Madison, Wisconsin, USA). 53705, programs: BLAST, BESTFIT, gap or PILEUP/PRETTYBOX). This type of software adapts identical or similar sequences by assigning degrees of homology to various substitutions, deletions and/or other modifications (substitution/scoring matrix: e.g., PAM, Blosum, GONET, JTT).

The term "modified amino acid sequence" refers to an amino acid sequence that compared to a reference sequence is modified in one or more amino acid positions. In particular in context of the present invention the term "modified SYNZIP amino acid sequence" shall refer to a SYNZIP sequence which is compared to a full-length sequence modified in one or more amino acid positions. A reference SYNZIP sequence is preferably a sequence shown in table 1 below. A preferred modified amino acid sequence is preferably a truncated amino acid sequence, in particular a modified SYNZIP amino acid sequence is a truncated SYNZIP amino acid sequence.

The term "truncated sequence" or "truncated protein" refers to an amino acid (protein) sequence that is shorter in length than a reference full length protein. In context of the invention a "truncated SYNZIP" refers to a sequence comprising a SYNZIP sequence that is not a full length known SYNZIP sequence (such as one of table 1 below) and that is shorter in length of such known SYNZIP sequence. Preferably, the truncated SYNZIP sequence when compared to the reference full length SYNZIP has truncation (removal) of a consecutive number of amino acids at either the C-terminus or the N-terminus (or both) of the reference full-length SYNZIP.

In **a first aspect,** the invention pertains to a synthetic fusion protein, comprising sequence elements (a), (b) and optionally (c), wherein:
**(a)** Is a non-ribosomal peptide synthetase (NRPS) or polyketide synthetase (PKS) amino acid sequence;
**(b)** Is a modified SYNZIP amino acid sequence; and
**(c)** Is a linker amino acid sequence which is in between (a) and (b).

In some preferred embodiments of the invention the synthetic fusion protein comprises the linker amino acid sequence, and wherein the linker amino acid sequence comprises, or consists essentially of, or consists of, one or more repeats of glycine and serine: (GS)ₙ, wherein n is a natural number from 1 to 15, preferably wherein n is 2 to 10, more preferably wherein n is 3, 4, 5, 6, 7, 8, 9, or 10, most preferably wherein n is 2, 3, 4 or 5.

As also defined above, in context of the invention preferred embodiments of the invention relates to a modified SYNZIP amino acid sequence which is a truncated SYNZIP amino acid sequence. More preferably an N-terminally truncated SYNZIP amino acid sequence.

A preferred embodiment of the invention pertains to "hybrid" complexes, which term shall be understood in context of the invention to relate to a combination of NRPS derived domains with PKS derived domains, preferably, using the present invention.

The synthetic fusion protein in accordance with the invention in some embodiments is a non-truncated SYNZIP amino acid sequence which is selected from a sequence of SYNZIP1 to SYNZIP 22 according to table 1:

**Table 1: Known prior art reference SYNZIP amino acid sequences**

| **Name** | **Amino Acid Sequence** | **SEQ ID NO:** |
|---|---|---|
| SYNZIP1 | NLVAQLENEVASLENENETLKKKNLHKKDLIAYLEKEIANLRKKIEE | 1 |
| SYNZIP2 | ARNAYLRKKIARLKKDNLQLERDEQNLEKIIANLRDEIARLENEVASHEQ | 2 |
| SYNZIP3 | NEVTTLENDAAFIENENAYLEKEIARLRKEKAALRNRLAHKK | 3 |
| SYNZIP4 | QKVAELKNRVAVKLNRNEQLKNKVEELKNRNAYLKNELATLENEVARL ENDVAE | 4 |
| SYNZIP5 | NTVKELKNYIQELEERNAELKNLKEHLKFAKAELEFELAAHKFE | 5 |
| SYNZIP6 | QKVAQLKNRVAYKLKENAKLENIVARLENDNANLEKDIANLEKDIANLE RDVAR | 6 |
| SYNZIP7 | KEIEYLEKEIERLKDLREHLKQDNAAHRQELNALRLEEAKLEFILAHLLS T | 7 |
| SYNZIP8 | KEIANLEKEIASLEKKVAVLKQRNAAHKQEVAALRKEIAYVEDEIQYVED E | 8 |
| SYNZIP9 | QKVESLKQKIEELKQRKAQLKNDIANLEKEIAYAET | 9 |
| SYNZIP1 0 | NLLATLRSTAAVLENENHVLEKEKEKLRKEKEQLLNKLEAYK | 10 |
| SYNZIP1 1 | ELTDELKNKKEALRKDNAALLNELASLENEIANLEKEIAYFK | 11 |
| SYNZIP1 2 | NEDLVLENRLAALRNENAALENDLARLEKEIAYLEKEIEREK | 12 |
| SYNZIP1 3 | QKVEELKNKIAELENRNAVKKNRVAHLKQEIAYLKDELAAHEFE | 13 |
| SYNZIP1 4 | NDLDAYEREAEKLEKKNEVLRNRLAALENELATLRQEVASMKQELQS | 14 |
| SYNZIP1 5 | FENVTHEFILATLENENAKLRRLEAKLERELARLRNEVAWL | 15 |
| SYNZIP1 6 | NILASLENKKEELKKLNAHLLKEIENLEKEIANLEKEIAYFK | 16 |
| SYNZIP1 7 | NEKEELKSKKAELRNRIEQLKQKREQLKQKIANLRKEIEAYK | 17 |
| SYNZIP1 8 | SIAATLENDLARLENENARLEKDIANLERDLAKLEREEAYF | 18 |
| SYNZIP1 9 | NELESLENKKEELKNRNEELKQKREQLKQKLAALRNKLDAYKNRL | 19 |
| SYNZIP2 0 | STVEELLRAIQELEKRNAELKNRKEELKNLVAHLRQELAAHKYE | 20 |
| SYNZIP2 1 | NEVAQLENDVAVIENENAYLEKEIARLRKEIAALRDRLAHKK | 21 |
| SYNZIP2 2 | KRIAYLRKKIAALKKDNANLEKDIANLENEIERLIKEIKTLENEVASHEQ | 22 |

In further preferred embodiments of the invention the modified synzip amino acid sequence when compared to one of the SYNZIP sequences in table 1 is truncated in 1 to 20, preferably 5 to 15, consecutive most N-terminal amino acids, preferably 6, 10 or 14 consecutive most N-terminal amino acids. Most preferably a modified SYNZIP sequence of the invention is a SYNZIP of table with an N-terminal 14 amino acid long (consecutive amino acids) truncation compared to the sequence of Table 1.

Preferred embodiments of the invention pertain to a synthetic fusion protein, wherein the modified synzip amino acid sequence is selected from any one of modified amino acid sequences of table 2.

**Table 2: modified SYNZIP sequences of the invention**

| **Synzip** | **N/C*** | **SEQ ID NO:** | **Amino Acid Sequence** |
|---|---|---|---|
| SZ1 | N | 23 | L VAQLENE VASLENE NETLKKK NLHKKDL IAYLEKE IANLRKK IEE |
| | | 24 | VAQLENE VASLENE NETLKKK NLHKKDL IAYLEKE IANLRKK IEE |
| | | 25 | AQLENE VASLENE NETLKKK NLHKKDL IAYLEKE IANLRKK IEE |
| | | 26 | QLENE VASLENE NETLKKK NLHKKDL IAYLEKE IANLRKK |
| | | | IEE |
| | | 27 | LENE VASLENE NETLKKK NLHKKDL IAYLEKE IANLRKK IEE |
| | | 28 | ENE VASLENE NETLKKK NLHKKDL IAYLEKE IANLRKK IEE (60 and 64) |
| | | 29 | NE VASLENE NETLKKK NLHKKDL IAYLEKE IANLRKK IEE |
| | | 30 | E VASLENE NETLKKK NLHKKDL IAYLEKE IANLRKK IEE |
| | | 31 | VASLENE NETLKKK NLHKKDL IAYLEKE IANLRKK IEE |
| | | 32 | ASLENE NETLKKK NLHKKDL IAYLEKE IANLRKK IEE |
| | | 33 | SLENE NETLKKK NLHKKDL IAYLEKE IANLRKK IEE |
| | | 34 | LENE NETLKKK NLHKKDL IAYLEKE IANLRKK IEE |
| | | 35 | ENE NETLKKK NLHKKDL IAYLEKE IANLRKK IEE |
| | | 36 | NE NETLKKK NLHKKDL IAYLEKE IANLRKK IEE (60 and 64) |
| | C | 37 | NL VAQLENE VASLENE NETLKKK NLHKKDL IAYLEKE IANLRKK IE |
| | | 38 | NL VAQLENE VASLENE NETLKKK NLHKKDL IAYLEKE IANLRKK I |
| | | 39 | NL VAQLENE VASLENE NETLKKK NLHKKDL IAYLEKE IANLRKK |
| | | 40 | NL VAQLENE VASLENE NETLKKK NLHKKDL IAYLEKE IANLRK |
| | | 41 | NL VAQLENE VASLENE NETLKKK NLHKKDL IAYLEKE IANLR |
| | | 42 | NL VAQLENE VASLENE NETLKKK NLHKKDL IAYLEKE IANL |
| | | 43 | NL VAQLENE VASLENE NETLKKK NLHKKDL IAYLEKE IAN |
| | | 44 | NL VAQLENE VASLENE NETLKKK NLHKKDL IAYLEKE IA |
| | | 45 | NL VAQLENE VASLENE NETLKKK NLHKKDL IAYLEKE I |
| | | 46 | NL VAQLENE VASLENE NETLKKK NLHKKDL IAYLEKE |
| | | 47 | NL VAQLENE VASLENE NETLKKK NLHKKDL IAYLEK |
| | | 48 | NL VAQLENE VASLENE NETLKKK NLHKKDL IAYLE |
| | | 49 | NL VAQLENE VASLENE NETLKKK NLHKKDL IAYL |
| | | 50 | NL VAQLENE VASLENE NETLKKK NLHKKDL IAY |
| SZ2 | N | 51 | R NAYLRKK IARLKKD NLQLERD EQNLEKI IANLRDE IARLENE VASHEQ |
| | | 52 | NAYLRKK IARLKKD NLQLERD EQNLEKI IANLRDE IARLENE VASHEQ |
| | | 53 | AYLRKK IARLKKD NLQLERD EQNLEKI IANLRDE IARLENE VASHEQ |
| | | 54 | YLRKK IARLKKD NLQLERD EQNLEKI IANLRDE IARLENE VASHEQ |
| | | 55 | LRKK IARLKKD NLQLERD EQNLEKI IANLRDE IARLENE VASHEQ |
| | | 56 | RKK IARLKKD NLQLERD EQNLEKI IANLRDE IARLENE VASHEQ |
| | | 57 | KK IARLKKD NLQLERD EQNLEKI IANLRDE IARLENE VASHEQ |
| | | 58 | K IARLKKD NLQLERD EQNLEKI IANLRDE IARLENE VASHEQ |
| | | 59 | IARLKKD NLQLERD EQNLEKI IANLRDE IARLENE VASHEQ (332 and 337) |
| | | 60 | ARLKKD NLQLERD EQNLEKI IANLRDE IARLENE VASHEQ (28 and 36) |
| | | 61 | RLKKD NLQLERD EQNLEKI IANLRDE IARLENE VASHEQ |
| | | 62 | LKKD NLQLERD EQNLEKI IANLRDE IARLENE VASHEQ |
| | | 63 | KKD NLQLERD EQNLEKI IANLRDE IARLENE VASHEQ |
| | | 64 | KD NLQLERD EQNLEKI IANLRDE IARLENE VASHEQ (28 and 36; 332 and 337) |
| | C | 65 | AR NAYLRKK IARLKKD NLQLERD EQNLEKI IANLRDE IARLENE VASHE |
| | | 66 | AR NAYLRKK IARLKKD NLQLERD EQNLEKI IANLRDE IARLENE VASH |
| | | 67 | AR NAYLRKK IARLKKD NLQLERD EQNLEKI IANLRDE IARLENE VAS |
| | | 68 | AR NAYLRKK IARLKKD NLQLERD EQNLEKI IANLRDE IARLENE VA |
| | | 69 | AR NAYLRKK IARLKKD NLQLERD EQNLEKI IANLRDE IARLENE V |
| | | 70 | AR NAYLRKK IARLKKD NLQLERD EQNLEKI IANLRDE IARLENE |
| | | 71 | AR NAYLRKK IARLKKD NLQLERD EQNLEKI IANLRDE IARLEN |
| | | 72 | AR NAYLRKK IARLKKD NLQLERD EQNLEKI IANLRDE IARLE |
| | | 73 | AR NAYLRKK IARLKKD NLQLERD EQNLEKI IANLRDE IARL |
| | | 74 | AR NAYLRKK IARLKKD NLQLERD EQNLEKI IANLRDE IAR |
| | | 75 | AR NAYLRKK IARLKKD NLQLERD EQNLEKI IANLRDE IA |
| | | 76 | AR NAYLRKK IARLKKD NLQLERD EQNLEKI IANLRDE I |
| | | 77 | AR NAYLRKK IARLKKD NLQLERD EQNLEKI IANLRDE |
| | | 78 | AR NAYLRKK IARLKKD NLQLERD EQNLEKI IANLRD |
| SZ3 | N | 79 | E VTTLEND AAFIENE NAYLEKE IARLRKE KAALRNR LAHKK |
| | | 80 | VTTLEND AAFIENE NAYLEKE IARLRKE KAALRNR LAHKK |
| | | 81 | TTLEND AAFIENE NAYLEKE IARLRKE KAALRNR LAHKK |
| | | 82 | TLEND AAFIENE NAYLEKE IARLRKE KAALRNR LAHKK |
| | | 83 | LEND AAFIENE NAYLEKE IARLRKE KAALRNR LAHKK |
| | | 84 | END AAFIENE NAYLEKE IARLRKE KAALRNR LAHKK |
| | | 85 | ND AAFIENE NAYLEKE IARLRKE KAALRNR LAHKK |
| | | 86 | D AAFIENE NAYLEKE IARLRKE KAALRNR LAHKK |
| | | 87 | AAFIENE NAYLEKE IARLRKE KAALRNR LAHKK |
| | | 88 | AFIENE NAYLEKE IARLRKE KAALRNR LAHKK |
| | | 89 | FIENE NAYLEKE IARLRKE KAALRNR LAHKK |
| | | 90 | IENE NAYLEKE IARLRKE KAALRNR LAHKK |
| | | 91 | ENE NAYLEKE IARLRKE KAALRNR LAHKK |
| | | 92 | NE NAYLEKE IARLRKE KAALRNR LAHKK |
| | C | 93 | NE VTTLEND AAFIENE NAYLEKE IARLRKE KAALRNR LAHK |
| | | 94 | NE VTTLEND AAFIENE NAYLEKE IARLRKE KAALRNR LAH |
| | | 95 | NE VTTLEND AAFIENE NAYLEKE IARLRKE KAALRNR LA |
| | | 96 | NE VTTLEND AAFIENE NAYLEKE IARLRKE KAALRNR L |
| | | 97 | NE VTTLEND AAFIENE NAYLEKE IARLRKE KAALRNR |
| | | 98 | NE VTTLEND AAFIENE NAYLEKE IARLRKE KAALRN |
| | | 99 | NE VTTLEND AAFIENE NAYLEKE IARLRKE KAALR |
| | | 100 | NE VTTLEND AAFIENE NAYLEKE IARLRKE KAAL |
| | | 101 | NE VTTLEND AAFIENE NAYLEKE IARLRKE KAA |
| | | 102 | NE VTTLEND AAFIENE NAYLEKE IARLRKE KA |
| | | 103 | NE VTTLEND AAFIENE NAYLEKE IARLRKE K |
| | | 104 | NE VTTLEND AAFIENE NAYLEKE IARLRKE |
| | | 105 | NE VTTLEND AAFIENE NAYLEKE IARLRK |
| | | 106 | NE VTTLEND AAFIENE NAYLEKE IARLR |
| SZ4 | N | 107 | K VAELKNR VAVKLNR NEQLKNK VEELKNR NAYLKNE LATLENE VARLEND VAE |
| | | 108 | VAELKNR VAVKLNR NEQLKNK VEELKNR NAYLKNE LATLENE VARLEND VAE |
| | | 109 | AELKNR VAVKLNR NEQLKNK VEELKNR NAYLKNE LATLENE VARLEND VAE |
| | | 110 | ELKNR VAVKLNR NEQLKNK VEELKNR NAYLKNE LATLENE VARLEND VAE |
| | | 111 | LKNR VAVKLNR NEQLKNK VEELKNR NAYLKNE LATLENE VARLEND VAE |
| | | 112 | KNR VAVKLNR NEQLKNK VEELKNR NAYLKNE LATLENE VARLEND VAE |
| | | 113 | NR VAVKLNR NEQLKNK VEELKNR NAYLKNE LATLENE VARLEND VAE |
| | | 114 | R VAVKLNR NEQLKNK VEELKNR NAYLKNE LATLENE VARLEND VAE |
| | | 115 | VAVKLNR NEQLKNK VEELKNR NAYLKNE LATLENE VARLEND VAE |
| | | 116 | AVKLNR NEQLKNK VEELKNR NAYLKNE LATLENE VARLEND VAE |
| | | 117 | VKLNR NEQLKNK VEELKNR NAYLKNE LATLENE VARLEND VAE |
| | | 118 | KLNR NEQLKNK VEELKNR NAYLKNE LATLENE VARLEND VAE |
| | | 119 | LNR NEQLKNK VEELKNR NAYLKNE LATLENE VARLEND VAE |
| | | 120 | NR NEQLKNK VEELKNR NAYLKNE LATLENE VARLEND VAE |
| | C | 121 | QK VAELKNR VAVKLNR NEQLKNK VEELKNR NAYLKNE LATLENE VARLEND VA |
| | | 122 | QK VAELKNR VAVKLNR NEQLKNK VEELKNR NAYLKNE LATLENE VARLEND V |
| | | 123 | QK VAELKNR VAVKLNR NEQLKNK VEELKNR NAYLKNE LATLENE VARLEND |
| | | 124 | QK VAELKNR VAVKLNR NEQLKNK VEELKNR NAYLKNE LATLENE VARLEN |
| | | 125 | QK VAELKNR VAVKLNR NEQLKNK VEELKNR NAYLKNE LATLENE VARLE |
| | | 126 | QK VAELKNR VAVKLNR NEQLKNK VEELKNR NAYLKNE LATLENE VARL |
| | | 127 | QK VAELKNR VAVKLNR NEQLKNK VEELKNR NAYLKNE LATLENE VAR |
| | | 128 | QK VAELKNR VAVKLNR NEQLKNK VEELKNR NAYLKNE LATLENE VA |
| | | 129 | QK VAELKNR VAVKLNR NEQLKNK VEELKNR NAYLKNE LATLENE V |
| | | 130 | QK VAELKNR VAVKLNR NEQLKNK VEELKNR NAYLKNE LATLENE |
| | | 131 | QK VAELKNR VAVKLNR NEQLKNK VEELKNR NAYLKNE LATLEN |
| | | 132 | QK VAELKNR VAVKLNR NEQLKNK VEELKNR NAYLKNE LATLE |
| | | 133 | QK VAELKNR VAVKLNR NEQLKNK VEELKNR NAYLKNE LATL |
| | | 134 | QK VAELKNR VAVKLNR NEQLKNK VEELKNR NAYLKNE LAT (21) |
| SZ5 | N | 135 | T VKELKNY IQELEER NAELKNL KEHLKFA KAELEFE LAAHKFE |
| | | 136 | VKELKNY IQELEER NAELKNL KEHLKFA KAELEFE LAAHKFE |
| | | 137 | KELKNY IQELEER NAELKNL KEHLKFA KAELEFE LAAHKFE |
| | | 138 | ELKNY IQELEER NAELKNL KEHLKFA KAELEFE LAAHKFE |
| | | 139 | LKNY IQELEER NAELKNL KEHLKFA KAELEFE LAAHKFE |
| | | 140 | KNY IQELEER NAELKNL KEHLKFA KAELEFE LAAHKFE |
| | | 141 | NY IQELEER NAELKNL KEHLKFA KAELEFE LAAHKFE |
| | | 142 | Y IQELEER NAELKNL KEHLKFA KAELEFE LAAHKFE |
| | | 143 | IQELEER NAELKNL KEHLKFA KAELEFE LAAHKFE |
| | | 144 | QELEER NAELKNL KEHLKFA KAELEFE LAAHKFE |
| | | 145 | ELEER NAELKNL KEHLKFA KAELEFE LAAHKFE |
| | | 146 | LEER NAELKNL KEHLKFA KAELEFE LAAHKFE |
| | | 147 | EER NAELKNL KEHLKFA KAELEFE LAAHKFE |
| | | 148 | ER NAELKNL KEHLKFA KAELEFE LAAHKFE |
| | C | 149 | NT VKELKNY IQELEER NAELKNL KEHLKFA KAELEFE LAAHKF |
| | | 150 | NT VKELKNY IQELEER NAELKNL KEHLKFA KAELEFE LAAHK |
| | | 151 | NT VKELKNY IQELEER NAELKNL KEHLKFA KAELEFE LAAH |
| | | 152 | NT VKELKNY IQELEER NAELKNL KEHLKFA KAELEFE LAA |
| | | 153 | NT VKELKNY IQELEER NAELKNL KEHLKFA KAELEFE LA |
| | | 154 | NT VKELKNY IQELEER NAELKNL KEHLKFA KAELEFE L |
| | | 155 | NT VKELKNY IQELEER NAELKNL KEHLKFA KAELEFE |
| | | 156 | NT VKELKNY IQELEER NAELKNL KEHLKFA KAELEF |
| | | 157 | NT VKELKNY IQELEER NAELKNL KEHLKFA KAELE |
| | | 158 | NT VKELKNY IQELEER NAELKNL KEHLKFA KAEL |
| | | 159 | NT VKELKNY IQELEER NAELKNL KEHLKFA KAE |
| | | 160 | NT VKELKNY IQELEER NAELKNL KEHLKFA KA |
| | | 161 | NT VKELKNY IQELEER NAELKNL KEHLKFA K |
| | | 162 | NT VKELKNY IQELEER NAELKNL KEHLKFA |
| SZ6 | N | 163 | K VAQLKNR VAYKLKE NAKLENI VARLEND NANLEKD IANLEKD IANLERD VAR |
| | | 164 | VAQLKNR VAYKLKE NAKLENI VARLEND NANLEKD IANLEKD IANLERD VAR |
| | | 165 | AQLKNR VAYKLKE NAKLENI VARLEND NANLEKD IANLEKD IANLERD VAR |
| | | 166 | QLKNR VAYKLKE NAKLENI VARLEND NANLEKD IANLEKD IANLERD VAR |
| | | 167 | LKNR VAYKLKE NAKLENI VARLEND NANLEKD IANLEKD IANLERD VAR |
| | | 168 | KNR VAYKLKE NAKLENI VARLEND NANLEKD IANLEKD IANLERD VAR |
| | | 169 | NR VAYKLKE NAKLENI VARLEND NANLEKD IANLEKD IANLERD VAR |
| | | 170 | R VAYKLKE NAKLENI VARLEND NANLEKD IANLEKD IANLERD VAR |
| | | 171 | VAYKLKE NAKLENI VARLEND NANLEKD IANLEKD IANLERD VAR |
| | | 172 | AYKLKE NAKLENI VARLEND NANLEKD IANLEKD IANLERD VAR |
| | | 173 | YKLKE NAKLENI VARLEND NANLEKD IANLEKD IANLERD VAR |
| | | 174 | KLKE NAKLENI VARLEND NANLEKD IANLEKD IANLERD VAR |
| | | 175 | LKE NAKLENI VARLEND NANLEKD IANLEKD IANLERD VAR |
| | | 176 | KE NAKLENI VARLEND NANLEKD IANLEKD IANLERD VAR |
| | C | 177 | QK VAQLKNR VAYKLKE NAKLENI VARLEND NANLEKD IANLEKD IANLERD VA |
| | | 178 | QK VAQLKNR VAYKLKE NAKLENI VARLEND NANLEKD IANLEKD IANLERD V |
| | | 179 | QK VAQLKNR VAYKLKE NAKLENI VARLEND NANLEKD IANLEKD IANLERD |
| | | 180 | QK VAQLKNR VAYKLKE NAKLENI VARLEND NANLEKD IANLEKD IANLER |
| | | 181 | QK VAQLKNR VAYKLKE NAKLENI VARLEND NANLEKD IANLEKD IANLE |
| | | 182 | QK VAQLKNR VAYKLKE NAKLENI VARLEND NANLEKD IANLEKD IANL |
| | | 183 | QK VAQLKNR VAYKLKE NAKLENI VARLEND NANLEKD IANLEKD IAN |
| | | 184 | QK VAQLKNR VAYKLKE NAKLENI VARLEND NANLEKD IANLEKD IA |
| | | 185 | QK VAQLKNR VAYKLKE NAKLENI VARLEND NANLEKD IANLEKD I |
| | | 186 | QK VAQLKNR VAYKLKE NAKLENI VARLEND NANLEKD IANLEKD |
| | | 187 | QK VAQLKNR VAYKLKE NAKLENI VARLEND NANLEKD IANLEK |
| | | 188 | QK VAQLKNR VAYKLKE NAKLENI VARLEND NANLEKD IANLE |
| | | 189 | QK VAQLKNR VAYKLKE NAKLENI VARLEND NANLEKD IANL |
| | | 190 | QK VAQLKNR VAYKLKE NAKLENI VARLEND NANLEKD IAN |
| SZ11 | N | 191 | L TDELKNK KEALRKD NAALLNE LASLENE IANLEKE IAYFK |
| | | 192 | TDELKNK KEALRKD NAALLNE LASLENE IANLEKE IAYFK |
| | | 193 | DELKNK KEALRKD NAALLNE LASLENE IANLEKE IAYFK |
| | | 194 | ELKNK KEALRKD NAALLNE LASLENE IANLEKE IAYFK |
| | | 195 | LKNK KEALRKD NAALLNE LASLENE IANLEKE IAYFK |
| | | 196 | KNK KEALRKD NAALLNE LASLENE IANLEKE IAYFK |
| | | 197 | NK KEALRKD NAALLNE LASLENE IANLEKE IAYFK |
| | | 198 | K KEALRKD NAALLNE LASLENE IANLEKE IAYFK |
| | | 199 | KEALRKD NAALLNE LASLENE IANLEKE IAYFK |
| | | 200 | EALRKD NAALLNE LASLENE IANLEKE IAYFK |
| | | 201 | ALRKD NAALLNE LASLENE IANLEKE IAYFK |
| | | 202 | LRKD NAALLNE LASLENE IANLEKE IAYFK |
| | | 203 | RKD NAALLNE LASLENE IANLEKE IAYFK |
| | | 204 | KD NAALLNE LASLENE IANLEKE IAYFK |
| | C | 205 | EL TDELKNK KEALRKD NAALLNE LASLENE IANLEKE IAYF |
| | | 206 | EL TDELKNK KEALRKD NAALLNE LASLENE IANLEKE IAY |
| | | 207 | EL TDELKNK KEALRKD NAALLNE LASLENE IANLEKE IA |
| | | 208 | EL TDELKNK KEALRKD NAALLNE LASLENE IANLEKE I |
| | | 209 | EL TDELKNK KEALRKD NAALLNE LASLENE IANLEKE |
| | | 210 | EL TDELKNK KEALRKD NAALLNE LASLENE IANLEK |
| | | 211 | EL TDELKNK KEALRKD NAALLNE LASLENE IANLE |
| | | 212 | EL TDELKNK KEALRKD NAALLNE LASLENE IANL |
| | | 213 | EL TDELKNK KEALRKD NAALLNE LASLENE IAN |
| | | 214 | EL TDELKNK KEALRKD NAALLNE LASLENE IA |
| | | 215 | EL TDELKNK KEALRKD NAALLNE LASLENE I |
| | | 216 | EL TDELKNK KEALRKD NAALLNE LASLENE |
| | | 217 | EL TDELKNK KEALRKD NAALLNE LASLEN |
| | | 218 | EL TDELKNK KEALRKD NAALLNE LASLE |
| SZ14 | N | 219 | D LDAYERE AEKLEKK NEVLRNR LAALENE LATLRQE VASMKQE LQS |
| | | 220 | LDAYERE AEKLEKK NEVLRNR LAALENE LATLRQE VASMKQE LQS |
| | | 221 | DAYERE AEKLEKK NEVLRNR LAALENE LATLRQE VASMKQE LQS |
| | | 222 | AYERE AEKLEKK NEVLRNR LAALENE LATLRQE VASMKQE LQS |
| | | 223 | YERE AEKLEKK NEVLRNR LAALENE LATLRQE VASMKQE LQS |
| | | 224 | ERE AEKLEKK NEVLRNR LAALENE LATLRQE VASMKQE LQS |
| | | 225 | RE AEKLEKK NEVLRNR LAALENE LATLRQE VASMKQE LQS |
| | | 226 | E AEKLEKK NEVLRNR LAALENE LATLRQE VASMKQE LQS |
| | | 227 | AEKLEKK NEVLRNR LAALENE LATLRQE VASMKQE LQS |
| | | 228 | EKLEKK NEVLRNR LAALENE LATLRQE VASMKQE LQS |
| | | 229 | KLEKK NEVLRNR LAALENE LATLRQE VASMKQE LQS |
| | | 230 | LEKK NEVLRNR LAALENE LATLRQE VASMKQE LQS |
| | | 231 | EKK NEVLRNR LAALENE LATLRQE VASMKQE LQS |
| | | 232 | KK NEVLRNR LAALENE LATLRQE VASMKQE LQS |
| | C | 233 | ND LDAYERE AEKLEKK NEVLRNR LAALENE LATLRQE VASMKQE LQ |
| | | 234 | ND LDAYERE AEKLEKK NEVLRNR LAALENE LATLRQE |
| | | | VASMKQE L |
| | | 235 | ND LDAYERE AEKLEKK NEVLRNR LAALENE LATLRQE VASMKQE |
| | | 236 | ND LDAYERE AEKLEKK NEVLRNR LAALENE LATLRQE VASMKQ |
| | | 237 | ND LDAYERE AEKLEKK NEVLRNR LAALENE LATLRQE VASMK |
| | | 238 | ND LDAYERE AEKLEKK NEVLRNR LAALENE LATLRQE VASM |
| | | 239 | ND LDAYERE AEKLEKK NEVLRNR LAALENE LATLRQE VAS |
| | | 240 | ND LDAYERE AEKLEKK NEVLRNR LAALENE LATLRQE VA |
| | | 241 | ND LDAYERE AEKLEKK NEVLRNR LAALENE LATLRQE V |
| | | 242 | ND LDAYERE AEKLEKK NEVLRNR LAALENE LATLRQE |
| | | 243 | ND LDAYERE AEKLEKK NEVLRNR LAALENE LATLRQ |
| | | 244 | ND LDAYERE AEKLEKK NEVLRNR LAALENE LATLR |
| | | 245 | ND LDAYERE AEKLEKK NEVLRNR LAALENE LATL |
| | | 246 | ND LDAYERE AEKLEKK NEVLRNR LAALENE LAT |
| SZ16 | N | 247 | I LASLENK KEELKKL NAHLLKE IENLEKE IANLEKE IAYFK |
| | | 248 | LASLENK KEELKKL NAHLLKE IENLEKE IANLEKE IAYFK |
| | | 249 | ASLENK KEELKKL NAHLLKE IENLEKE IANLEKE IAYFK |
| | | 250 | SLENK KEELKKL NAHLLKE IENLEKE IANLEKE IAYFK |
| | | 251 | LENK KEELKKL NAHLLKE IENLEKE IANLEKE IAYFK |
| | | 252 | ENK KEELKKL NAHLLKE IENLEKE IANLEKE IAYFK |
| | | 253 | NK KEELKKL NAHLLKE IENLEKE IANLEKE IAYFK |
| | | 254 | K KEELKKL NAHLLKE IENLEKE IANLEKE IAYFK |
| | | 255 | KEELKKL NAHLLKE IENLEKE IANLEKE IAYFK |
| | | 256 | EELKKL NAHLLKE IENLEKE IANLEKE IAYFK |
| | | 257 | ELKKL NAHLLKE IENLEKE IANLEKE IAYFK |
| | | 258 | LKKL NAHLLKE IENLEKE IANLEKE IAYFK |
| | | 259 | KKL NAHLLKE IENLEKE IANLEKE IAYFK |
| | | 260 | KL NAHLLKE IENLEKE IANLEKE IAYFK |
| | C | 261 | NI LASLENK KEELKKL NAHLLKE IENLEKE IANLEKE IAYF |
| | | 262 | NI LASLENK KEELKKL NAHLLKE IENLEKE IANLEKE IAY |
| | | 263 | NI LASLENK KEELKKL NAHLLKE IENLEKE IANLEKE IA |
| | | 264 | NI LASLENK KEELKKL NAHLLKE IENLEKE IANLEKE I |
| | | 265 | NI LASLENK KEELKKL NAHLLKE IENLEKE IANLEKE |
| | | 266 | NI LASLENK KEELKKL NAHLLKE IENLEKE IANLEK |
| | | 267 | NI LASLENK KEELKKL NAHLLKE IENLEKE IANLE |
| | | 268 | NI LASLENK KEELKKL NAHLLKE IENLEKE IANL |
| | | 269 | NI LASLENK KEELKKL NAHLLKE IENLEKE IAN |
| | | 270 | NI LASLENK KEELKKL NAHLLKE IENLEKE IA |
| | | 271 | NI LASLENK KEELKKL NAHLLKE IENLEKE I |
| | | 272 | NI LASLENK KEELKKL NAHLLKE IENLEKE |
| | | 273 | NI LASLENK KEELKKL NAHLLKE IENLEK |
| | | 274 | NI LASLENK KEELKKL NAHLLKE IENLE |
| SZ17 | N | 275 | EKEELKSKKAELRNRIEQLKQKREQLKQKIANLRKEIEAYK |
| | | 276 | KEELKSKKAELRNRIEQLKQKREQLKQKIANLRKEIEAYK |
| | | 277 | EELKSKKAELRNRIEQLKQKREQLKQKIANLRKEIEAYK |
| | | 278 | ELKSKKAELRNRIEQLKQKREQLKQKIANLRKEIEAYK |
| | | 279 | LKSKKAELRNRIEQLKQKREQLKQKIANLRKEIEAYK |
| | | 280 | KSKKAELRNRIEQLKQKREQLKQKIANLRKEIEAYK |
| | | 281 | SKKAELRNRIEQLKQKREQLKQKIANLRKEIEAYK (281, 288, 295) |
| | | 282 | KKAELRNRIEQLKQKREQLKQKIANLRKEIEAYK |
| | | 283 | KAELRNRIEQLKQKREQLKQKIANLRKEIEAYK |
| | | 284 | AELRNRIEQLKQKREQLKQKIANLRKEIEAYK |
| | | 285 | ELRNRIEQLKQKREQLKQKIANLRKEIEAYK |
| | | 286 | LRNRIEQLKQKREQLKQKIANLRKEIEAYK |
| | | 287 | RNRIEQLKQKREQLKQKIANLRKEIEAYK |
| | | 288 | NRIEQLKQKREQLKQKIANLRKEIEAYK (281, 288, 295) |
| | C | 289 | N EKEELKSKKAELRN RI EQLKQKREQLKQKIAN LRKEI EA Y |
| | | 290 | N EKEELKSKKAELRN RI EQLKQKREQLKQKIAN LRKEI EA |
| | | 291 | NEKEELKSKKAELRNRIEQLKQKREQLKQKIANLRKEIE |
| | | 292 | NEKEELKSKKAELRNRIEQLKQKREQLKQKIANLRKEI |
| | | 293 | NEKEELKSKKAELRNRIEQLKQKREQLKQKIANLRKE |
| | | 294 | NEKEELKSKKAELRNRIEQLKQKREQLKQKIANLRK |
| | | 295 | NEKEELKSKKAELRNRIEQLKQKREQLKQKIANLR (281, 288, 295) |
| | | 296 | NEKEELKSKKAELRNRIEQLKQKREQLKQKIANL |
| | | 297 | NEKEELKSKKAELRNRIEQLKQKREQLKQKIAN |
| | | 298 | NEKEELKSKKAELRNRIEQLKQKREQLKQKIA |
| | | 299 | NEKEELKSKKAELRNRIEQLKQKREQLKQKI |
| | | 300 | NEKEELKSKKAELRNRIEQLKQKREQLKQK |
| | | 301 | NEKEELKSKKAELRNRIEQLKQKREQLKQ |
| | | 302 | NEKEELKSKKAELRNRIEQLKQKREQLK |
| SZ18 | N | 303 | I AATLEND LARLENE NARLEKD IANLERD LAKLERE EAYF |
| | | 304 | AATLEND LARLENE NARLEKD IANLERD LAKLERE EAYF |
| | | 305 | ATLEND LARLENE NARLEKD IANLERD LAKLERE EAYF |
| | | 306 | TLEND LARLENE NARLEKD IANLERD LAKLERE EAYF |
| | | 307 | LEND LARLENE NARLEKD IANLERD LAKLERE EAYF |
| | | 308 | END LARLENE NARLEKD IANLERD LAKLERE EAYF |
| | | 309 | ND LARLENE NARLEKD IANLERD LAKLERE EAYF (309, 316, 324) |
| | | 310 | D LARLENE NARLEKD IANLERD LAKLERE EAYF |
| | | 311 | LARLENE NARLEKD IANLERD LAKLERE EAYF |
| | | 312 | ARLENE NARLEKD IANLERD LAKLERE EAYF |
| | | 313 | RLENE NARLEKD IANLERD LAKLERE EAYF |
| | | 314 | LENE NARLEKD IANLERD LAKLERE EAYF |
| | | 315 | ENE NARLEKD IANLERD LAKLERE EAYF |
| | | 316 | NE NARLEKD IANLERD LAKLERE EAYF (309, 316, 324) |
| | C | 317 | SI AATLEND LARLENE NARLEKD IANLERD LAKLERE EAY |
| | | 318 | SI AATLEND LARLENE NARLEKD IANLERD LAKLERE EA |
| | | 319 | SI AATLEND LARLENE NARLEKD IANLERD LAKLERE E |
| | | 320 | SI AATLEND LARLENE NARLEKD IANLERD LAKLERE |
| | | 321 | SI AATLEND LARLENE NARLEKD IANLERD LAKLER |
| | | 322 | SI AATLEND LARLENE NARLEKD IANLERD LAKLE |
| | | 323 | SI AATLEND LARLENE NARLEKD IANLERD LAKL |
| | | 324 | SI AATLEND LARLENE NARLEKD IANLERD LAK (309, 316, 324) |
| | | 325 | SI AATLEND LARLENE NARLEKD IANLERD LA |
| | | 326 | SI AATLEND LARLENE NARLEKD IANLERD L |
| | | 327 | SI AATLEND LARLENE NARLEKD IANLERD |
| | | 328 | SI AATLEND LARLENE NARLEKD IANLER |
| | | 329 | SI AATLEND LARLENE NARLEKD IANLE |
| | | 330 | SI AATLEND LARLENE NARLEKD IANL |
| SZ19 | N | 331 | E LESLENK KEELKNR NEELKQK REQLKQK LAALRNK LDAYKNR L |
| | | 332 | LESLENK KEELKNR NEELKQK REQLKQK LAALRNK LDAYKNR L (59 and 64) |
| | | 333 | [?] ESLENK KEELKNR NEELKQK REQLKQK LAALRNK LDAYKNR L |
| | | 334 | SLENK KEELKNR NEELKQK REQLKQK LAALRNK LDAYKNR L |
| | | 335 | LENK KEELKNR NEELKQK REQLKQK LAALRNK LDAYKNR L |
| | | 336 | ENK KEELKNR NEELKQK REQLKQK LAALRNK LDAYKNR L |
| | | 337 | NK KEELKNR NEELKQK REQLKQK LAALRNK LDAYKNR L (59 and 64) |
| | | 338 | K KEELKNR NEELKQK REQLKQK LAALRNK LDAYKNR L |
| | | 339 | KEELKNR NEELKQK REQLKQK LAALRNK LDAYKNR L |
| | | 340 | EELKNR NEELKQK REQLKQK LAALRNK LDAYKNR L |
| | | 341 | ELKNR NEELKQK REQLKQK LAALRNK LDAYKNR L |
| | | 342 | LKNR NEELKQK REQLKQK LAALRNK LDAYKNR L |
| | | 343 | KNR NEELKQK REQLKQK LAALRNK LDAYKNR L |
| | | 344 | NR NEELKQK REQLKQK LAALRNK LDAYKNR L |
| | C | 345 | NE LESLENK KEELKNR NEELKQK REQLKQK LAALRNK LDAYKNR |
| | | 346 | NE LESLENK KEELKNR NEELKQK REQLKQK LAALRNK LDAYKN |
| | | 347 | NE LESLENK KEELKNR NEELKQK REQLKQK LAALRNK LDAYK |
| | | 348 | NE LESLENK KEELKNR NEELKQK REQLKQK LAALRNK LDAY |
| | | 349 | NE LESLENK KEELKNR NEELKQK REQLKQK LAALRNK LDA |
| | | 350 | NE LESLENK KEELKNR NEELKQK REQLKQK LAALRNK LD |
| | | 351 | NE LESLENK KEELKNR NEELKQK REQLKQK LAALRNK L |
| | | 352 | NE LESLENK KEELKNR NEELKQK REQLKQK LAALRNK |
| | | 353 | NE LESLENK KEELKNR NEELKQK REQLKQK LAALRN |
| | | 354 | NE LESLENK KEELKNR NEELKQK REQLKQK LAALR |
| | | 355 | NE LESLENK KEELKNR NEELKQK REQLKQK LAAL |
| | | 356 | NE LESLENK KEELKNR NEELKQK REQLKQK LAA |
| | | 357 | NE LESLENK KEELKNR NEELKQK REQLKQK LA |
| | | 358 | NE LESLENK KEELKNR NEELKQK REQLKQK L |
| SZ20 | N | 359 | T VEELLRA IQELEKR NAELKNR KEELKNL VAHLRQE LAAHKYE |
| | | 360 | VEELLRA IQELEKR NAELKNR KEELKNL VAHLRQE LAAHKYE |
| | | 361 | EELLRA IQELEKR NAELKNR KEELKNL VAHLRQE LAAHKYE |
| | | 362 | ELLRA IQELEKR NAELKNR KEELKNL VAHLRQE LAAHKYE |
| | | 363 | LLRA IQELEKR NAELKNR KEELKNL VAHLRQE LAAHKYE |
| | | 364 | LRA IQELEKR NAELKNR KEELKNL VAHLRQE LAAHKYE |
| | | 365 | RA IQELEKR NAELKNR KEELKNL VAHLRQE LAAHKYE |
| | | 366 | A IQELEKR NAELKNR KEELKNL VAHLRQE LAAHKYE |
| | | 367 | IQELEKR NAELKNR KEELKNL VAHLRQE LAAHKYE |
| | | 368 | QELEKR NAELKNR KEELKNL VAHLRQE LAAHKYE |
| | | 369 | ELEKR NAELKNR KEELKNL VAHLRQE LAAHKYE |
| | | 370 | LEKR NAELKNR KEELKNL VAHLRQE LAAHKYE |
| | | 371 | EKR NAELKNR KEELKNL VAHLRQE LAAHKYE |
| | | 372 | KR NAELKNR KEELKNL VAHLRQE LAAHKYE |
| | C | 373 | ST VEELLRA IQELEKR NAELKNR KEELKNL VAHLRQE LAAHKY |
| | | 374 | ST VEELLRA IQELEKR NAELKNR KEELKNL VAHLRQE LAAHK |
| | | 375 | ST VEELLRA IQELEKR NAELKNR KEELKNL VAHLRQE LAAH |
| | | 376 | ST VEELLRA IQELEKR NAELKNR KEELKNL VAHLRQE LAA |
| | | 377 | ST VEELLRA IQELEKR NAELKNR KEELKNL VAHLRQE LA |
| | | 378 | ST VEELLRA IQELEKR NAELKNR KEELKNL VAHLRQE L |
| | | 379 | ST VEELLRA IQELEKR NAELKNR KEELKNL VAHLRQE |
| | | 380 | ST VEELLRA IQELEKR NAELKNR KEELKNL VAHLRQ |
| | | 381 | ST VEELLRA IQELEKR NAELKNR KEELKNL VAHLR |
| | | 382 | ST VEELLRA IQELEKR NAELKNR KEELKNL VAHL |
| | | 383 | ST VEELLRA IQELEKR NAELKNR KEELKNL VAH |
| | | 384 | ST VEELLRA IQELEKR NAELKNR KEELKNL VA |
| | | 385 | ST VEELLRA IQELEKR NAELKNR KEELKNL V |
| | | 386 | ST VEELLRA IQELEKR NAELKNR KEELKNL |
| SZ21 | N | 387 | E VAQLEND VAVIENE NAYLEKE IARLRKE IAALRDR LAHKK |
| | | 388 | VAQLEND VAVIENE NAYLEKE IARLRKE IAALRDR LAHKK |
| | | 389 | AQLEND VAVIENE NAYLEKE IARLRKE IAALRDR LAHKK |
| | | 390 | QLEND VAVIENE NAYLEKE IARLRKE IAALRDR LAHKK |
| | | 391 | LEND VAVIENE NAYLEKE IARLRKE IAALRDR LAHKK |
| | | 392 | END VAVIENE NAYLEKE IARLRKE IAALRDR LAHKK |
| | | 393 | ND VAVIENE NAYLEKE IARLRKE IAALRDR LAHKK |
| | | 394 | D VAVIENE NAYLEKE IARLRKE IAALRDR LAHKK |
| | | 395 | VAVIENE NAYLEKE IARLRKE IAALRDR LAHKK |
| | | 396 | AVIENE NAYLEKE IARLRKE IAALRDR LAHKK |
| | | 397 | VIENE NAYLEKE IARLRKE IAALRDR LAHKK |
| | | 398 | IENE NAYLEKE IARLRKE IAALRDR LAHKK |
| | | 399 | ENE NAYLEKE IARLRKE IAALRDR LAHKK |
| | | 400 | NE NAYLEKE IARLRKE IAALRDR LAHKK |
| | C | 401 | NE VAQLEND VAVIENE NAYLEKE IARLRKE IAALRDR LAHK |
| | | 402 | NE VAQLEND VAVIENE NAYLEKE IARLRKE IAALRDR LAH |
| | | 403 | NE VAQLEND VAVIENE NAYLEKE IARLRKE IAALRDR LA |
| | | 404 | NE VAQLEND VAVIENE NAYLEKE IARLRKE IAALRDR L |
| | | 405 | NE VAQLEND VAVIENE NAYLEKE IARLRKE IAALRDR |
| | | 406 | NE VAQLEND VAVIENE NAYLEKE IARLRKE IAALRD |
| | | 407 | NE VAQLEND VAVIENE NAYLEKE IARLRKE IAALR |
| | | 408 | NE VAQLEND VAVIENE NAYLEKE IARLRKE IAA |
| | | 409 | NE VAQLEND VAVIENE NAYLEKE IARLRKE IA |
| | | 410 | NE VAQLEND VAVIENE NAYLEKE IARLRKE I |
| | | 411 | NE VAQLEND VAVIENE NAYLEKE IARLRKE |
| | | 412 | NE VAQLEND VAVIENE NAYLEKE IARLRK |
| | | 413 | NE VAQLEND VAVIENE NAYLEKE IARLR |
| | | 414 | NE VAQLEND VAVIENE NAYLEKE IARL |

| | | | |
|---|---|---|---|
| *N or C indicates N- or C-terminal location of truncation | | | |

In some additional or alternative embodiments, invention provides the synthetic fusion protein described herein, wherein the fusion protein is in N- to C-terminal direction (a), optionally (c), and (b); or (b), optionally (c), and (a).

In some additional or alternative embodiments, the invention provides the synthetic fusion protein described herein, wherein the fusion protein is in N- to C-terminal direction (a), optionally (c), and (b); and wherein (a) comprises an adenylation domain as most C-terminal NRPS or PKS domain sequence.

In some additional or alternative embodiments, the invention provides the synthetic fusion protein described herein, wherein the fusion protein is in N- to C-terminal direction (b), optionally (c), and (a), and wherein the N terminal domain sequence of (a) is a thiolation domain.

In some additional or alternative embodiments, the invention provides the synthetic fusion protein described herein, wherein the fusion protein is in N- to C-terminal direction (a), optionally (c), and (b); and wherein (a) comprises a thiolation domain as most C-terminal NRPS.

In some additional or alternative embodiments, the invention provides the synthetic fusion protein described herein, wherein the fusion protein is in N- to C-terminal direction (b), optionally (c), and (a), and wherein the N terminal domain sequence of (a) is a condensation domain.

In some additional or alternative embodiments, the invention provides the synthetic fusion protein described herein, wherein the fusion protein is in N- to C-terminal direction (a), optionally (c), and (b); and wherein (a) comprises a condensation domain as most C-terminal NRPS domain sequence;

In some additional or alternative embodiments, the invention provides the synthetic fusion protein described herein, wherein the fusion protein is in N- to C-terminal direction (b), optionally (c), and (a), and wherein the N terminal domain sequence of (a) is an adenylation domain.

Preferred modified SYNZIPs of the invention as shown in Table 2 above are shown in SEQ ID NO: 23-414.

In most preferred embodiments, the synthetic fusion protein described herein does not comprise any one of the full lengths SYNZIP1 to SYNZIP 22 of table 1 (in the description).

Preferred modified SYNZIPs present invention are such sequences having a truncation of at least 10 amino acids. Therefore, preferred modified SYNZPS are selected from SEQ ID Nos: 32, 33, 34, 35, 36, 46, 47, 48, 49, 50, 60, 61, 62, 63, 64, 74, 75, 76, 77, 78, 88, 89, 90, 91, 92, 102, 103, 104, 105, 106, 116, 117, 118, 119, 120, 130, 131, 132, 133, 134, 144, 145, 146, 147, 148, 158, 159, 160, 161, 162, 172, 173, 174, 175, 176, 186, 187, 188, 189, 190, 200, 201, 202, 203, 204, 214, 215, 216, 217, 218, 228, 229, 230, 231, 232, 242, 243, 244, 245, 246, 256, 257, 258, 259, 260, 270, 271, 272, 273, 274, 284, 285, 286, 287, 288, 298, 299, 300, 301, 302, 312, 313, 314, 315, 316, 326, 327, 328, 329, 330, 340, 341, 342, 343, 344, 354, 355, 356, 357, 358, 368, 369, 370, 371, 372, 382, 383, 384, 385, 386, 396, 397, 398, 399, 400, 410, 411, 412, 413, and 414.

Even more preferably, the modified SYNZIP of the invention are truncated with 14 amino acids. Such modified SYNZIPS having a 14 amino acid truncation are selected from: SEQ ID Nos: 36, 50, 64, 78, 92, 106, 120, 134, 148, 162, 176, 190, 204, 218, 232, 246, 260, 274, 288, 302, 316, 330, 344, 358, 372, 386, 400, and 414.

In **a second aspect,** the invention pertains to an assembly system of synthetic NRPS or PKS complexes, comprising at least two synthetic fusion protein of the first aspect, wherein at least one of the at least two fusion protein is a donor synthetic fusion protein and is in N- to C-terminal direction (a), optionally (c), and (b); and wherein at least one of the at least two fusion proteins is an acceptor synthetic fusion protein and is in N- to C-terminal direction (b), optionally (c), and (a).

In a preferred embodiment, the invention provides the assembly system, wherein the donor synthetic fusion protein comprises a modified SYNZIP amino acid sequence that is capable of specifically interacting (binding) with the modified SYNZIP amino acid sequence of the acceptor synthetic fusion protein (SYNZIP pair).

In **a third aspect,** the invention pertains to a synthetic NRPS or PKS complex, wherein at least two NRPS or PKS domains are connected via a non-covalent interaction between two modified SYNZIP amino acid sequences.

The invention in a preferred embodiment of this aspect pertains to a synthetic NRPS or PKS complex, wherein the two modified SYNZIP amino acid sequences are truncated SYNZIP amino acid sequences, preferably as recited and defined in the above first and second aspects of the invention.

The synthetic NRPS or PKS complex of the invention is preferred, wherein the modified synzip amino acid sequence is connected to a domain of a NRPS or PKS via a linker sequence, wherein the linker amino acid sequence comprises, or consists essentially of, or consists of, one or more repeats of glycine and serine: (GS)n, wherein n is a natural number from 1 to 15, preferably wherein n is 2 to 10, more preferably wherein n is 3, 4, 5, 6, 7, 8, 9, or 10, most preferably wherein n is 2, 3, 4 or 5.

A preferred embodiment of this aspect pertains to complexes comprising two modified SYNZIP amino acid sequences (a modified SYNZIP pair) is selected from the following pairs (A and B) of modified SYNZIP amino acid sequences of the invention. The combinations of A and B are provided in tables 3 and 4 below.: [

**Table 3: preferred modified SYNZIP pairs of the invention**

| **A** | **B** |
|---|---|
| 28 | 60 |
| 28 | 64 |
| 36 | 60 |
| 36 | 64 |
| 59 | 332 |
| 59 | 337 |
| 64 | 332 |
| 64 | 337 |
| 281 | 281 |
| 281 | 288 |
| 281 | 295 |
| 288 | 288 |
| 288 | 295 |
| 295 | 295 |
| 309 | 309 |
| 309 | 316 |
| 309 | 324 |
| 316 | 316 |
| 316 | 324 |
| 324 | 324 |

**Table 4: particularly preferred pairs of modified SYNZIPs:**

| **A** | **B** |
|---|---|
| 28 | 60 |
| 28 | 64 |
| 36 | 60 |
| 36 | 64 |
| 59 | 332 |
| 59 | 337 |
| 64 | 332 |
| 64 | 337 |

In **a fourth aspect,** the invention pertains to a nucleic acid encoding a synthetic fusion protein of the first aspect.

In preferred embodiments of the invention the nucleic acids of the invention are provided as nucleic acid constructs, for example for the expression of the encoded protein.

The term "nucleic acid construct" is herein referred to as a nucleic acid molecule, either single- or double-stranded, which is isolated from a naturally occurring gene or which has been modified to contain segments of nucleic acid which are combined and juxtaposed in a manner which would not otherwise exist in nature. The term nucleic acid construct is synonymous with the term "expression cassette" when the nucleic acid construct contains all the control sequences required for expression of a coding sequence, wherein said control sequences are operably linked to said coding sequence.

The term "operably linked" is defined herein as a configuration in which a control sequence is appropriately placed at a position relative to the coding sequence of the DNA sequence such that the control sequence directs the production of a polypeptide.

The term "control sequences" is defined herein to include all components, which are necessary or advantageous for the expression of mRNA and/or a polypeptide, either in vitro or in a host cell. Each control sequence may be native or foreign to the nucleic acid sequence encoding the polypeptide. Such control sequences include, but are not limited to, a leader, Shine-Delgarno sequence, optimal translation initiation sequences (as described in Kozak, 1991, J. Biol. Chem. 266:19867-19870), a polyadenylation sequence, a pro-peptide sequence, a pre-pro-peptide sequence, a promoter, a signal sequence, and a transcription terminator. At a minimum, the control sequences include a promoter, and transcriptional and translational stop signals. Control sequences may be optimized to their specific purpose. Preferred optimized control sequences used in the present invention are those described in WO2006/077258, which is herein incorporated by reference.

The control sequences may be provided with linkers for the purpose of introducing specific restriction sites facilitating ligation of the control sequences with the coding region of the nucleic acid sequence encoding a polypeptide. The control sequence may be an appropriate promoter sequence (promoter). The control sequence may also be a suitable transcription terminator (terminator) sequence, a sequence recognized by a filamentous fungal cell to terminate transcription. The terminator sequence is operably linked to the 3'-terminus of the nucleic acid sequence encoding the polypeptide. Any terminator, which is functional in the cell, may be used in the present invention.

The control sequence may also be a polyadenylation sequence, a sequence which is operably linked to the 3'-terminus of the nucleic acid sequence and which, when transcribed, is recognized by the filamentous fungal cell as a signal to add polyadenosine residues to transcribed mRNA. Any polyadenylation sequence, which is functional in the cell, may be used in the present invention.

The term "promoter" is defined herein as a DNA sequence that binds RNA polymerase and directs the polymerase to the correct downstream transcriptional start site of a nucleic acid sequence encoding a biological compound to initiate transcription. RNA polymerase effectively catalyzes the assembly of messenger RNA complementary to the appropriate DNA strand of a coding region. The term "promoter" will also be understood to include the 5'-non-coding region (between promoter and translation start) for translation after transcription into mRNA, cis-acting transcription control elements such as enhancers, and other nucleotide sequences capable of interacting with transcription factors. The promoter may be any appropriate promoter sequence suitable for a eukaryotic or prokaryotic host cell, which shows transcriptional activity, including mutant, truncated, and hybrid promoters, and may be obtained from polynucleotides encoding extra-cellular or intracellular polypeptides either homologous (native) or heterologous (foreign) to the cell. The promoter may be a constitutive or inducible promoter.

In **a fifth aspect,** the invention pertains to a library of nucleic acid sequences, wherein the library comprises two or more nucleic acids, and wherein the two or more nucleic acids comprised in the library encode the assembly system of synthetic NRPS or PKS complexes of any one of the preceding aspects.

In **a sixth aspect,** the invention pertains to a method of assembling a complete or partial synthetic NRPS or PKS, the method comprising bringing into contact, or expressing together, at least a donor synthetic fusion protein and an acceptor synthetic fusion protein of any one of the preceding claims.

The terms "of the [present] invention", "in accordance with the invention", "according to the invention" and the like, as used herein are intended to refer to all aspects and embodiments of the invention described and/or claimed herein.

As used herein, the term "comprising" is to be construed as encompassing both "including" and "consisting of", both meanings being specifically intended, and hence individually disclosed embodiments in accordance with the present invention. Where used herein, "and/or" is to be taken as specific disclosure of each of the two specified features or components with or without the other. For example, "A and/or B" is to be taken as specific disclosure of each of (i) A, (ii) B and (iii) A and B, just as if each is set out individually herein. In the context of the present invention, the terms "about" and "approximately" denote an interval of accuracy that the person skilled in the art will understand to still ensure the technical effect of the feature in question. The term typically indicates deviation from the indicated numerical value by ±20%, ±15%, ±10%, and for example ±5%. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. Where an indefinite or definite article is used when referring to a singular noun, e.g. "a", "an" or "the", this includes a plural of that noun unless something else is specifically stated.

It is to be understood that application of the teachings of the present invention to a specific problem or environment, and the inclusion of variations of the present invention or additional features thereto (such as further aspects and embodiments), will be within the capabilities of one having ordinary skill in the art in light of the teachings contained herein.

Unless context dictates otherwise, the descriptions and definitions of the features set out above are not limited to any particular aspect or embodiment of the invention and apply equally to all aspects and embodiments which are described.

All references, patents, and publications cited herein are hereby incorporated by reference in their entirety.

### BRIEF DESCRIPTION OF THE FIGURES

The figures show:
**Figure 1****:** (taken from Reinke et al. J. AM. CHEM. SOC. 2010, 132, 6025-6031; incorporated herein in its entirety) shows the SYNZIP interaction partners and possible networks. Protein microarray assay results of 26 peptides forming specific interactive pairs. Peptides which are immobilised on the surface of the microarray are shown in series. Fluorescence-labelled peptides in solution are listed in row. According to the array score (shown on the right), black spots show a strong (0-0.2) and white spots a weak fluorescence signal (>1.0). The absence of homospecific interactions is indicated by the red diagonal line. Interactions that showed an array score of ≤ 0.2 are highlighted in green. The number of strong interaction partners is shown in the lower column..

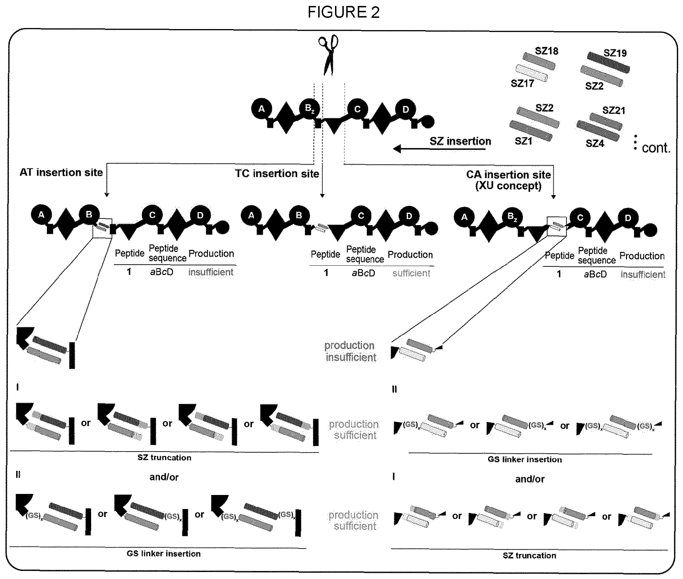

**Figure 2****:** shows the optimization of type S NRPSs. From a pool of 27 biophysically characterized pairs Synzips (SZs) can be introduced in three different positions within the A-T, T-C and C-A linker regions, respectively. For type S NRPSs yielding insufficient production titers, the inventors explored two optimization strategies: (I) the truncation of SZs either from the C-terminal, N-terminal or both sides; (2) the insertion of flexible GS linkers upstream of the SZ donor, downstream of the SZ acceptor or into both positions.

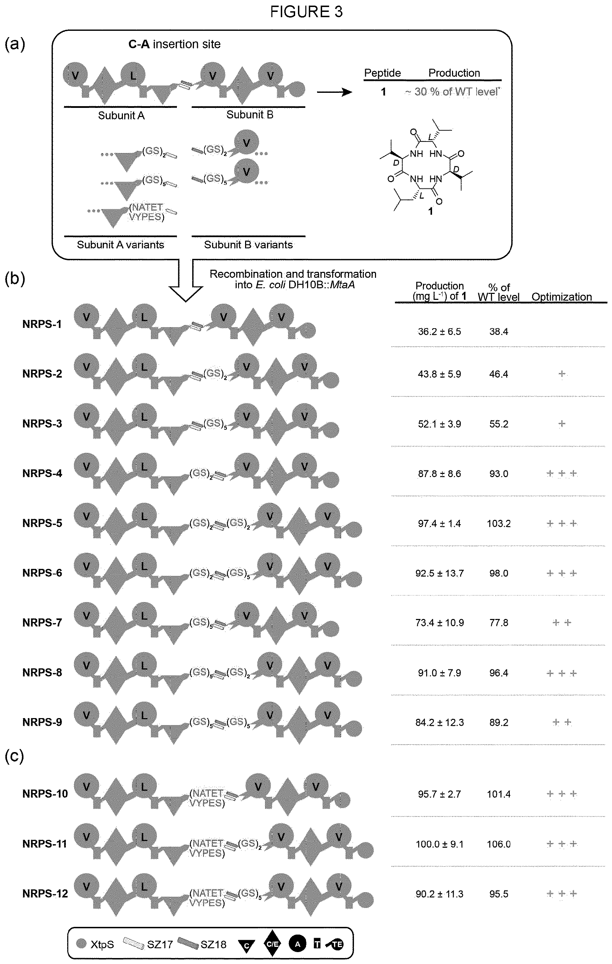

**Figure 3****:** shows the insertion of synthetic GS-stretches of varying length into a chimeric XtpS system. (a) Between each experimental approach, the production of non-optimized NRPS-1 varies, but is on average at -30% of WT level. A set of modified subunit A and B variants were constructed by inserting GS-stretches of 4-10 AAs between the C-terminus of XtpS subunit A and SZ17 and SZ18 and the N-terminus of subunit B. (b) Generated modified subunits were re-combined with non-modified subunits and transformed into *E. coli* DH10B::mtaA to obtain NRPS-2 to -9. (c) The native 10 AA (NATETVYPES) were additionally interested between the C-terminus of subunit A and SZ17. Production titres of NRPS-1 to -12 were compared with each other and rated from - - - - - , - -, -, to O, +, ++, +++. Corresponding peptide yields (mg/L) and standard deviations are obtained from biological triplicate experiments. For domain assignment, the following symbols are used: (A, large circles), (T, rectangle), (C, triangle), (C/E, diamond), (TE, small circle); substrate specificities are assigned for all A domains and indicated by capital letters.

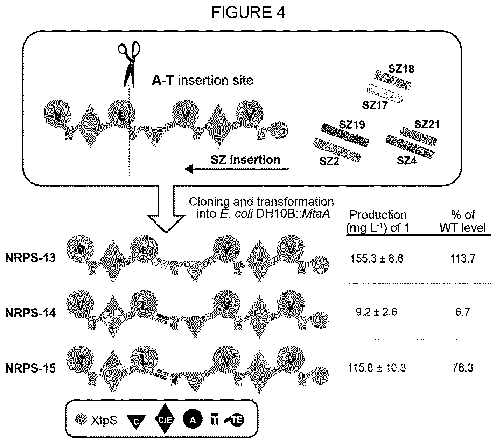

**Figure 4****:** shows the introduction of other SZ pairs into XtpS. Two parallel interacting SZs SZ2:19 and SZ4:21 were introduced into the A-T position of module two to generate NRPS-14 and NRPS-15. Corresponding peptide yields (mg/L) and standard deviations are obtained from biological triplicate experiments. Domain assignment is as described before.

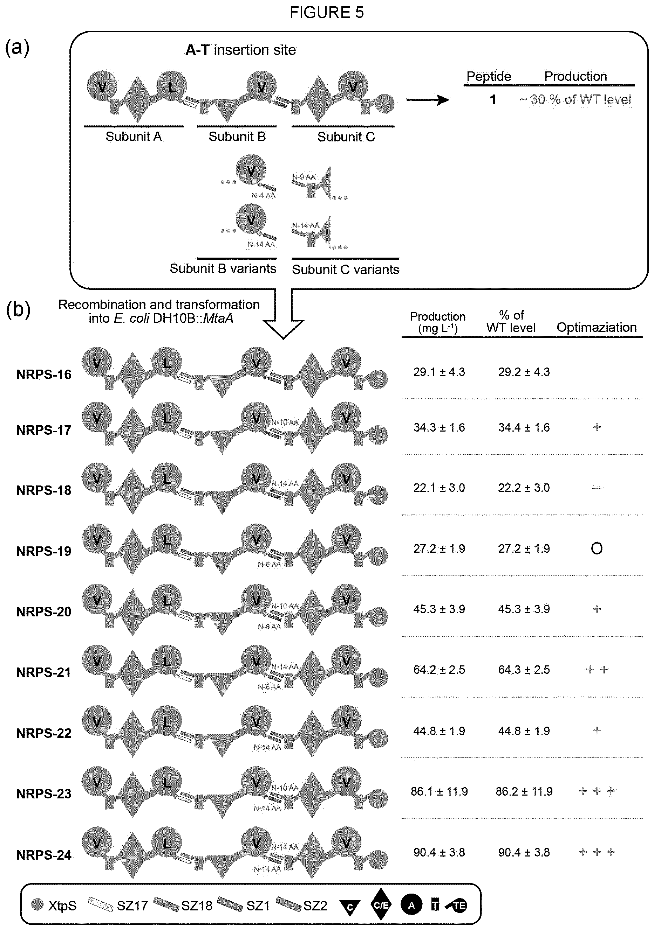

**Figure 5****:** shows the truncation of SZ1:2 in the tri-partite XtpS. Between each experimental approach, the production of non-optimized NRPS-16 varies, but is on average at -30% of WT level. A set of modified subunit B and C variants were constructed by *N*-terminally truncating SZ1 by 6 AAs and 14 AAs and SZ2 by 10 AAs and 14 AAs. Generated modified subunits were re-combined with non-modified subunits and transformed into *E. coli* DH10B::*MtaA* to obtain NRPS-16 to -24. Corresponding peptide yields (mg/L) and standard deviations are obtained from biological triplicate experiments. Rating of production titres and domain assignment is as described before.

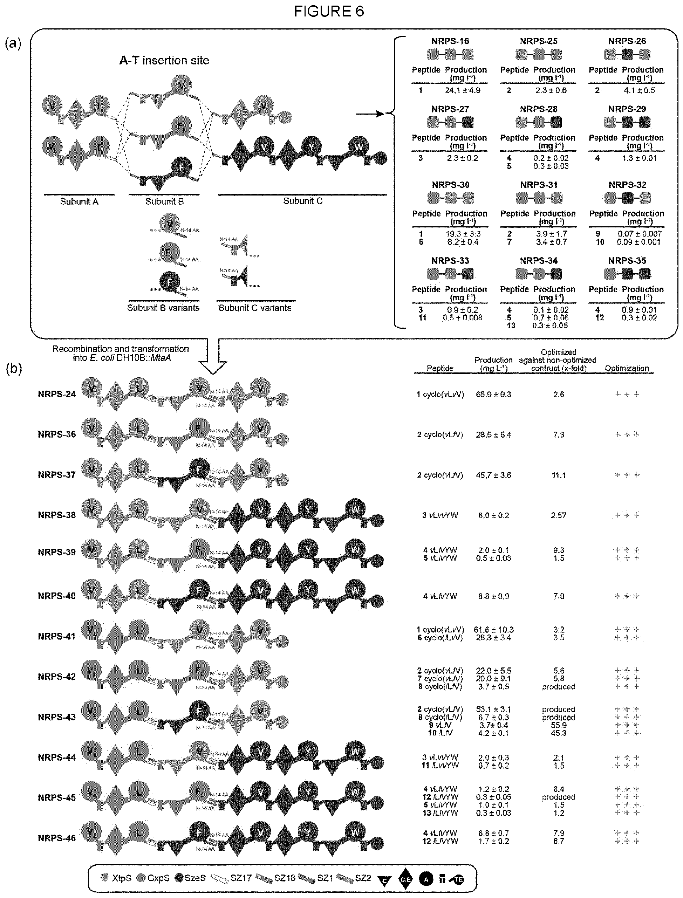

**Figure 6****:** shows the optimized Tri-partite NRPS library. Production titres of non-optimized constructs NRPS-16 and NRPS-36 to-46 are shown on the upper right corner. Subunits B and C were modified by removing 14 AAs from the N-terminal site of SZ1 and SZ2. Generated modified subunits were re-combined with non-modified subunits A and transformed into *E. coli* DH10B::*mtaA* to obtain NRPS-24 to -46. Production titres of NRPS-24 to -46 were compared with non-optimized NRPS-15 to -35 and rated with from - ,- - , - - , to O, +, ++, +++. Corresponding peptide yields (mg/L) and standard deviations are obtained from biological triplicate experiments. Domain assignment is as described before.

### EXAMPLES

Certain aspects and embodiments of the invention will now be illustrated by way of example and with reference to the description, figures and tables set out herein. Such examples of the methods, uses and other aspects of the present invention are representative only, and should not be taken to limit the scope of the present invention to only such representative examples.

The examples show:

### Example 1: Insertion of GS Linker Sequences

The inventors introduced the antiparallel SZ pair 17 & 18 (SZ17:18) at three different positions within the A-T, T-C and C-A linker regions of the xenotetrapeptide producing synthetase (XtpS) from *Xenorhabdus nematophila* ATCC 19061,21 to create three different two protein type S XtpS variants. Although all variants showed catalytic activity, synthesizing the expected cyclic xenotetrapeptide (1, cyclo (vLvV)), the inventors also found that the type S XtpS split within the C-A linker region (c.f., NRPS-1, Figure 3), resulted in low production of 1 with -30% compared to WT level (Figure 3a), while both type S XtpS splits within the A-T and T-C linker regions produced 1 at -86% compared to WT XtpS level. Of note, throughout the present work, mentioned recombinant type S and WT NRPSs were produced heterologously in *E. coli* DH10B::mtaA. The resulting peptides and yields were confirmed by HPLC-MS/MS and comparison of retention times with synthetic standards (c.f., Supporting Information Figures S3-24).

In order to improve expression, the inventors introduced synthetic Gly-Ser (GS) segments of 4 and10 AAs in length between the C-terminus of NRPS-1 subunit A (subA_GS0) and SZ17 (subA_GS2, subA_GS5) as well as SZ18 and the N-terminus of NRPS-1 subunit B (sub_GS0, sub_GS2, sub_GS5), respectively (Fig. 3a).

To test the devised modifications the inventors co-transformed, produced, and analyzed all possible type S subA:subB combinations (NRPS-2 to -9) and compared peptide yields of 1 with the previously created unmodified type S NRPS-1 (subA_GS0:subB_GS0) and WT XtpS. In brief, all modified type S NRPSs (NRPS-2 to -9) showed increased catalytic activity compared to NRPS-1 (38 mg L-1) producing 1 at titers of 44 - 100 mg L-1. Interestingly, while insertions of GS2 (NRPS-2; 46 mg L-1) and GS5 (NRPS-3; 55 mg L-1) stretches downstream of SZ18 (subB_GS2 & sub_GS5), respectively, let to the least increase in peptide production, any modification upstream of SZ17 (subA_GS2, subA_GS5) significantly raised production titers of 1 close to (NRPS-7; 78 mg L-1) or back to (NRPS-4; 88 mg L-1) WT level.

Additionally, to run a control experiment, the inventors reinserted the original 10 AAs (NATETVYPES (SEQ ID NO: 415) of the C-A linker which were deleted in an original feasibility study in order to maintain the native spacing of the C and A domains involved. As the inventors identified the position upstream of SZ17 as structurally more suitable, the inventors choose this position to reinsert the original AAs into NRPS-1 subunit A (subA_NATETVYPES) shifting the original XU fusion site by 10 AAs. From the three additionally generated synthetases harboring subA_NATETVYPES (NRPS-10 to -12, Fig 2c), NRPS-11 was the best producing synthetase resulting from a combination of subA_NATETVYPES with subB_GS2 (100 mg L-1), biosynthesizing 1 at 106% compared to WT XtpS and 278% compared to NRPS-1.

Further modified type S XtpS with GS2 and GS5 linker insertions were done (NRPS-49 to -56). Here, GS stretches were introduced into a tri-partite type S XtpS system (depicted in, Fig. 5, NRPS-16) partitioned in between the A2-T2 and A3-T3 linker regions by introducing SZ17:18 and SZ1:2 pairs, respectively (NRPS-16) - with emphasis on optimizing the latter SZ pair facilitating the specific interaction of the tripartite NRPS's subunits B and C. Interestingly, again all created type S assembly lines (NRPS-49 to -56) showed catalytic activity with yields ranging from 30 to 52 mg L-1 and all but two (NRPS-51 & -49) showed slightly increased amounts of 1 compared to unmodified type S NRPS-16, but still only at 25 % compared to its dipartite counterpart (Fig. 4, NRPS-13). Further improvement was deemed helpful.

### Example 2: Introduction of other SZ pairs

Depending on the experimental approach, it might be necessary to introduce additional or other SZ pairs than the previously applied pairs SZ17:18 and SZ1:2. Based on the observed mixed effects of the previously used SZ pairs on the productivity of type S NRPSs, the inventors decided to evaluate the effects of further SZ pairs on the functionality and productivity of NRPSs. Again targeting XtpS, we introduced two additional parallel interacting SZ pairs, SZ2:19 (NRPS-14) and SZ21:4 (NRPS-15) at the A-T position within module two (Fig. 4).

While both NRPS were functional, NRPS-14 (10 mg L-1) and -15 (115 mg L-1) resulted in reduced biosynthesis of 1 compared to WT XtpS (136 mg L-1) and NRPS-13 (155 mg L-1) (Fig. 4), the impairing effects of SZ2:19 appeared to be significantly greater. Since the experimental setup for NRPS-13 to -15 was identical except for the selected SZ pair, it is obvious that the respective selected SZ pairs are responsible for the observed effects on peptide yields.

The inventors then took a closer look at the biophysical data of all SZs used, compiled in a specification sheet provided by Thompson et al. The inventors found that all three SZ pairs have similar properties, e.g. similar affinities of <10 nm and non-toxicity to the living cell (demonstrated by yeast-two-hybrid experiments with two different reporter genes), but they considerably differ in length.20 Here, SZ17:18 used in the best producing NRPS-13 harbors the shortest SZ pair with a length of 42 AAs (SZ17) and 41 AAs (SZ18), respectively. All other SZs available and tested so far are significantly longer, ranging from 45 AAs to 54 AAs. These insights combined the results of GS linker modifications of SZ1:2, prompted the inventors to infer a direct correlation between the length of SZs and the productivity of type S NRPSs. Consequently, we next planned to optimize chimeric type S NRPSs by truncating applied SZs.

### Example 3: Truncation of SYNZIPs

The possibility of generating tri-partite type S assembly lines that were assembled from three independent type S subunits was demonstrated. Although type S tri-partite NRPSs further enhance the advantages associated with the insertion of SZs, i.e. increased biocombinatorial potential, they have so far suffered from low production titers (-30% of WT level, c.f. NRPS-16 Fig. 5) compared to their WT and di-partite counterparts (-86% of WT level, c.f. Fig. S2). Thus, to optimize these systems we again targeted the SZ pair 1:2 of NRPS-16 (Fig. 5) - which was already target of our GS linker insertion strategy but resulted in moderate optimization results.

As a rational to guide the optimization attempt of the invention, the inventors found that the N-terminal truncation of SZ4 had no effect on its stability, while the C-terminal truncation noticeably destabilized SZ4. Consequently, it was decided to N-terminally truncate both, SZ1 (-6 and -14 AAs) and SZ2 (-10 and -14 AAs) attached to subunit B and subunit C, respectively, of NRPS-16. Co-production of all modified and unmodified subunit B and C variants together with unmodified subunit A resulted in NRPS-17 to NRPS-24 (Fig. 5). Of note, whereas the SZ pair SZ1_-6_AAs and SZ2_-10_AAs was created to simulate the length of SZ17:18, the SZ pair SZ1_-14_AAs and SZ2_-14_AAa was created because for SZ4 the two most N-terminal heptads were described as dispensable.

All resulting assembly lines showed catalytic activity biosynthesizing 1 in a range of 22 - 90 mg L-1. Of these, all but NRPS-18 and -19 resulted in a 17% to 210% increase in 1 compared to NRPS-16, with NRPS-23 (86 mg L-1) and -24 (90 mg L-1) showing the highest titers almost restoring WT XtpS production, highlighting the great potential of truncating SZs for type S NRPS optimization. However, to test the effects of further, more invasive truncations, the inventors also attempted to remove 28 AAs from the N-terminus of SZ1 and 2, but found that the synthesis of 1 decreased to 62% compared to WT XtpS (NPRS-57), suggesting that the ideal truncation is probably in the range of 14 AAs. More truncations were done (see table 2). In brief, truncation of SZ2:19 in NRPS-14 resulted in an increased production of four constructs (NRPS-58, -61, -63 and -64) with NRPS-64 even restoring the synthesis of 1 to WT levels ( NRPS-58 to -65).

Lastly, the inventors applied the identified best performing SZ1:2 variant to the tri-partite SZ library, to determine whether the observed product yield-increasing changes are exclusively linked to Xtps type S assembly line variants or whether there is an observable generality in this approach. To this end the inventors modified all functional (12 out of 18) type S NRPSs subunit B and subunit C variants by removing 14 AAs from the N-terminal sites of SZ1 and SZ2, respectively. As shown in Fig. 6, all optimized type S NRPSs resulted in a strong increase in production ranging from 123.2% to 5592.4% compared to the non-optimized constructs. These results not only confirm the clear correlation between the length of SZs and the productivity of type S NRPSs, but also inferred the general applicability of this particular optimized SZ pair for the generation of high-yield artificial type S assembly lines rather than being exclusively tied to a particular NRPS.

In conclusion, apparently by truncating SZs, preferably by 14 N-terminal amino acids, their disturbing effects can be completely abolished, allowing SZs to be used without any restrictions for the generation of reprogrammed NRPSs.

## Claims

1. **A synthetic fusion protein,** comprising sequence elements (a), (b) and optionally (c), wherein:
(a) Is a non-ribosomal peptide synthetase (NRPS) or a polyketide synthetase (PKS) amino acid sequence;
(b) Is a modified SYNZIP amino acid sequence; and
(c) Is a linker amino acid sequence which is in between (a) and (b).

2. The synthetic fusion protein of claim 1, comprising the linker amino acid sequence, and wherein the linker amino acid sequence comprises, or consists essentially of, or consists of, one or more repeats of glycine and serine: (GS)ₙ, wherein n is a natural number from 1 to 15, preferably wherein n is 2 to 10, more preferably wherein n is 3, 4, 5, 6, 7, 8, 9, or 10, most preferably wherein n is 2, 3, 4 or 5.

3. The synthetic fusion protein of claim 1 or 2, wherein the modified SYNZIP amino acid sequence, is a truncated SYNZIP amino acid sequence.

4. The synthetic fusion protein of any one of claims 1 to 3, wherein the modified SYNZIP amino acid sequence, is a N-terminally truncated SYNZIP amino acid sequence.

5. The synthetic fusion protein of any one of claims 1 to 4, wherein a non-truncated SYNZIP amino acid sequence is selected from a SYNZIP1 to SYNZIP 22 according to **Table 1.**

6. The synthetic fusion protein of claim 5, wherein the modified SYNZIP amino acid sequence when compared to one of the SYNZIP sequences in table 1 is truncated in 1 to 20, preferably 5 to 15, most N-terminal amino acids, preferably 6, 10 or 14 most N-terminal amino acids.

7. The synthetic fusion protein of claim 6, wherein the modified SYNZIP amino acid sequence is selected from any one of modified amino acid sequences of **Table 2.**

8. **An assembly system of synthetic NRPS or PKS complexes,** comprising at least two synthetic fusion protein of any one of claims 1 to 7, wherein at least one of the at least two fusion protein is a donor synthetic fusion protein and is in N- to C-terminal direction (a), optionally (c), and (b); and wherein at least one of the at least two fusion proteins is an acceptor synthetic fusion protein and is in N- to C-terminal direction (b), optionally (c), and (a).

9. The assembly system of claim 8, wherein the donor synthetic fusion protein comprises a modified SYNZIP amino acid sequence that is capable of specifically interacting (binding) with the modified SYNZIP amino acid sequence of the acceptor synthetic fusion protein (SYNZIP pair).

10. **A synthetic NRPS or PKS complex,** wherein at least two NRPS or PKS domains are connected via a non-covalent interaction between two modified SYNZIP amino acid sequences.

11. The synthetic NRPS or PKS of claim 10, wherein the two modified SYNZIP amino acid sequences are truncated SYNZIP amino acid sequences as recited in any one of the preceding claims, preferably, wherein the two modified SYNZIP amino acids are a pair of A and B as indicated in table 3 or table 4.

12. The synthetic NRPS or PKS of claim 10 or 11, wherein the modified SYNZIP amino acid sequence is connected to a domain of a NRPS or PKS via a linker sequence, wherein the linker amino acid sequence comprises, or consists essentially of, or consists of, one or more repeats of glycine and serine: (GS)ₙ, wherein n is a natural number from 1 to 15, preferably wherrein n is 2 to 10, more preferably wherein n is 3, 4, 5, 6, 7, 8, 9, or 10, most preferably wherein n is 2, 3, 4 or 5.

13. **A nucleic acid** encoding a synthetic fusion protein of any one of claims 1 to 7.

14. **A library of nucleic acid sequences**, wherein the library comprises two or more nucleic acids, and wherein the two or more nucleic acids comprised in the library encode the assembly system of synthetic NRPS or PKS complexes of any one of the preceding claims.

15. **A method of assembling a complete or partial synthetic NRPS or PKS**, the method comprising bringing into contact, or expressing together, at least a donor synthetic fusion protein and an acceptor synthetic fusion protein of any one of the preceding claims.
